# NEUE EUROPÄISCHE PATENTSCHRIFT

(11) **EP 0 773 008 B2**
(45) Veröffentlichungstag und Bekanntmachung des Hinweises auf die Entscheidung über den Einspruch: **20.05.2009**
(45) Hinweis auf die Patenterteilung: 11.02.2004
(21) Anmeldenummer: 95810701.3
(22) Anmeldetag: 08.11.1995
(51) Int. Cl.: A61F 2/44, A61F 2/46, A61B 17/32

(54) **Zwischenwirbelprothese**
Intervertebral prosthesis
Prothèse intervertébrale

(43) Veröffentlichungstag der Anmeldung: 14.05.1997
(60) Teilanmeldung: 03013559.4
(73) Patentinhaber: Zimmer GmbH, 8404 Winterthur (CH)
(72) Erfinder: Husson, Jean-Louis, Prof. Dr., F-35000 Rennes (FR); Baumgartner, Walter, CH-9500 Wil (CH); Freudiger, Stefan, CH-3047 Bremgarten (CH)
(74) Vertreter: Manitz, Finsterwald & Partner GbR

(56) Entgegenhaltungen:
- EP-A- 0 321 884
- EP-A- 0 453 393
- EP-A- 0 621 020
- EP-A- 0 700 671
- FR-A- 2 712 486
- US-A- 3 448 737
- US-A- 5 171 281
- US-A- 5 176 647
- US-A- 5 263 927

## Beschreibung

Die Erfindung betrifft ein Zwischenwirbelimplantat bestehend aus einem länglichen, elastischen Körper. Eine Prothese dieser Art ist aus EP-A-0 453 393 bekannt. Sie dient zum Ersatz eines beschädigten Bandscheibenkernes (nucleus pulposus). Dabei bleibt der äussere Ring (anulus fibrosus) einer natürlichen Bandscheibe weitgehend erhalten. Die bekannte Prothese besteht aus einem flüssigkeitsdichten Hohlkörper, der mit einem inkompressiblen, fliessfähigen Medium gefüllt ist. Diese Prothese benötigt zu ihrer Implantation ein besonders ausgebildetes Einführungsinstrument, mit dem der Hohlkörper intra-operativ zu Spiralform aufgewickelt und anschliessend gefüllt werden kann. Dies stellt hohe Anforderungen an die Geschicklichkeit des Operateurs einerseits, sowie an die Herstellung des Einführungsinstruments hinsichtlich des Aufwickelmechanismus, sowie hinsichtlich der Füllvorrichtung. Ferner stellen die Dichtheitskriterien des Hohlkörpers in Anbetracht der hohen Belastungen im Wirbelsäulenbereich auf Dauer ein Risiko dar. Nachdem der Körper im Zwischenwirbelbereich aufgerollt und gefüllt worden ist, liegt das freie Ende am anulus fibrosus an, falls das Hilfswerkzeug, welches zum Füllen und Abdichten im Zentrum der Spirale angreift, entfernbar ist ohne Hohlräume zu erzeugen. Nur wenn kein Abwandern von Spiralenteilen in andere Hohlräume erfolgen kann, können die bei Belastung auftretenden, radial gerichteten Kräfte kompensiert werden und die Spirale erhält die gewünschte Stützfunktion.

In der FR-A-2 712 486 werden spiralförmige Implantate für den Kernbereich einer Bandscheibe gezeigt, die aus Formgedächtnis-Legierungen (memorymetal) bestehen, welche sich auf Grund eines physikalischen Phänomens aus einem spannungsfreien Zustand in eine andere Form mit spannungsfreiem Zustand deformieren. Als physikalisches Phänomen werden das Auftreten einer Zugspannung und ein Wechsel der Temperatur aufgeführt, welche die ursprünglichen physikalischen Bedingungen verändern. Andere Beispiele zeigen häkchenbesetzte Transportbänder, die durch ein Rohr gezogen werden, um ein mitgeschlepptes, häkchenbesetztes Band zu einer Spirale aufzuwickeln.

US 3,867,728 beschreibt u.a. ein Zwischenwirbelimplantat in Form einer ovalen Scheibe, wobei diese Form durch variieren der Dicke eines aufgewickelden länglichen Körpers erreicht wird.

Aufgabe der Erfindung ist es, ein Implantat zu schaffen, das eine einfache Handhabung erlaubt.

Diese Aufgabe wird gelöst durch ein Implantat gemäss Anspruch 1. Der Querschnitt des Körpers ist vorzugsweise rechteckig, wobei die Kanten auch abgerundet sein können. Es ist auch möglich Körper mit rundlichem Querschnitt zu verwenden, solange bei Belastung eine im wesentlichen formschlüssige, verkantungssichere Lage der Spirale erhalten bleibt. Der Querschnitt des elastischen Körpers ist nicht konstant über seine ganze Länge, vielmehr nimmt die Dicke nach aussen hin ab. Eine derartige Spirale hat den Vorteil, dass sie sich selbst ablegt, beim Einstossen nicht knickt und in einer passenden aufgewickelten Länge abtrennbar ist.

Das Implantat kann als solches die Form einer Zylinderscheibe annehmen, also flach ausgebildet sein, oder aber den Konturen der Wirbelflächen folgend Wölbungen nach aussen und/oder innen aufweisen. Dadurch wird erreicht, dass sich die bei Belastung erzeugten Kräfte gleichmässiger auf die Wirbelflächen verteilen. Der Durchmesser der Spirale ist vorzugsweise so bemessen, dass der Raum innerhalb des anulus fibrosus ausgefüllt wird.

Um die Bandscheibe einzuführen, hat es sich als vorteilhaft erwiesen, Versteifungsmittel in Form eines Kunststoff- oder Metallbandes am freien Ende der Spirale anzubringen, über welches eine Abtrennvorrichtung bis an eine vorgesehene Trennstelle geführt werden kann. Das Band weist im kräftefreien Zustand vorzugsweise keine Krümmung auf. Nach erfolgter Implantation wird das Band zusammen mit dem nicht benötigten Spiralenanteil von der abgelegten Spirale mittels der Abtrennvorrichtung abgetrennt.

Um die Lage des Implantates intra- und post-operativ kontrollieren zu können, empfiehlt sich das Vorsehen von röntgen-positiven Markierungen in Form von Kugeln oder eines Fadens auf bzw. entlang innerhalb des elastischen Körpers.

Das Material, aus dem der elastische Körper hergestellt ist, entspricht im wesentlichen den elastischen Eigenschaften einer Bandscheibe, sodass die auftretenden Kräfte möglichst gleichmässig auf die Wirbelfläche übertragen werden. Als vorteilhaft hat sich die Verwendung von Polyurethan oder Polyurethan-Silicongemischen herausgestellt, welche eine Shore-Härte im Bereich von etwa 80A aufweisen. Verwendet werden können auch Schäume verschiedenster Kunststoffe, sofern sie den elastischen Anforderungen genügen.

In einer besonders bevorzugten Ausführung weist die Spriale ein zentrales Mittelstück auf, um welches sich die weiteren Windungen dicht anlegen. Es entstehen nur kleine Zwischenräume, die sich gegebenenfalls im implantierten Zustand mit Bandscheibenflüssigkeit füllen und die ein Verkanten des elastischen Körpers unter Radialkräften nicht zulassen. Durch ein geeignetes Höhenzu Breitenverhältnis wird ein Verkanten des elastischen Körpers generell verhindert. Das Mittelstück kann grundsätzlich eine kreiszylindrische Form aufweisen, aber auch Formen wie Kugel oder Ellipsoid haben sich als nützlich erwiesen. Daran schliesst sich vorteilhafter Weise ein kurzer, dünnerer Übergangsteil, über den die Verbindung zur weiteren Spirale herstellt ist. Dies hat den Vorteil, dass die Spirale in dichtester, flächenschlüssiger Packung verbleibt und die grösstmögliche Flächenverteilung unter Abbau von Kraftspitzen erreicht wird. In einer einfacheren Ausgestaltung des Implantates kann das zentrale Mittelstück auch weggelassen werden, da sein Flächenbeitrag im Verhältnis zur Gesamtfläche klein ist.

Die Höhe der Spirale entspricht im wesentlichen dem physiologischen Zwischenwirbelabstand. Aus den elastischen Eigenschaften des verwendeten Materials ergibt sich die bevorzugte Dicke, in der der elastische Körper auszubilden ist. An der Peripherie der Spirale, nach etwa drei bis fünf Windungen, je nach Material und Breite der Windung, kann die Dicke des Körpers abnehmen, da die anliegenden Kräfte primär zentral aufgenommen werden sollen und da andererseits der Körper an entsprechender Stelle abtrennbar sein soll. In einer weiteren Ausgestaltung der Erfindung ist es möglich, die Höhe des Implantates den individuellen Wirbelanständen anzupassen.

Das Abtrennen soll vorzugsweise innerhalb des Hohlraumes erfolgen, wofür die entsprechenden Kräfte mittels eines noch näher zu beschreibenden Instrumentes aufgebracht werden müssen.

Hiermit wird auch ein Verfahren zur Herstellung eines Zwischenwirbelimplantates in Spiralform beschrieben. Als Herstellungs Verfahren wird ein Spritzgussverfahren eingesetzt, wobei die schneckenförmige Gussform im Spiralinneren angegossen wird. Das so erhaltene Implantat weist eine Spiralform mit stark formschlüssigen Windungen auf.

Weiter wird eine Vorrichtung zum Einbringen des Implantates beschrieben, welche im wesentlichen ein Rohr mit rechteckigem Querschnitt aufweist, in dem der elastische Körper eingezogen werden kann und zwar vorzugsweise so weit, dass das Mittelstück noch aus dem distalen Ende des Rohres ragt. Der Querschnitt des Rohres entspricht im wesentlichen dem Querschnitt des elastischen Körpers. Der Vorschub des Körpers kann von Hand erfolgen. Vorzugsweise sind jedoch unter entsprechender Querschnittsvergrösserung des Rohres Vorschubmittel in Form eines Stössels vorgesehen, welche z.B. über einen Hebelmechanismus aktiviert werden können. Der Stössel weist an seinem distalen, in Wirkverbindung mit dem elastischen Körper oder der Versteifung stehenden Ende Mittel auf, die einen Vorschub des Körpers erleichtern. Die Mittel können darin bestehen, die Oberfläche aufzurauhen, oder aber auch in zahnartigen Fortsätzen mit Vorzugsorientierung in distaler Richtung. Dadurch wird erreicht, dass die Fortsätze bei Vorschub des Stössels in den elastischen Körper oder in die Versteifung eingreifen und ihn/sie mit sich schieben, während sie bei Rückzug des Stössels über die Oberfläche nach hinten gleiten. Dadurch ist ein kontrollierter Vorschub des elastischen Körpers möglich. Von zusätzlichem Vorteil ist es, das Rohr an seinem distalen Ende mit einer einseitigen Querschnittsverengung auszugestalten, welche an ihrem schmalen Ende nur noch den elastischen Körper aufnehmen kann. Dies bewirkt, dass der Stössel beim Vorschieben gleichzeitig nach unten gedrückt wird und so den Kraftschluss zwischen Stössel und Körper verstärkt.

Das Rohr der Einbringvorrichtung kann vorteilhafter Weise auch gekrümmt sein, da das Einbringen des Implantates in den Zwischenwirbelraum vorzugsweise von seitlich dorsal erfolgt. Die Krümmung kann sowohl nach oben oder unten, sowie wahlweise nach links oder rechts ausgeführt sein, je nachdem von welcher Seite der Zugang erfolgen soll. Dabei kann die Einbringvorrichtung auch vorne keilförmig auslaufen, um benachbarte Wirbelkörper nach entstandenem Abstandsverlust wieder ausreichend mit dem keilförmigen Teil zu distanzieren.

Beschrieben wird ferner eine Vorrichtung zum Abtrennen des nicht benötigten Spiralenendes. Nachdem die Zwischenwirbelprothese implantiert worden ist, wird die Einführvorrichtung entlang der Versteifung, welche wenigstens teilweise ausserhalb des Bandscheibenbereichs verbleibt, zurückgezogen und das Abtrenninstrument aufgeschoben. Dieses Instrument weist vorzugsweise einen Wirkmechanismus ähnlich einer Zange auf, umfassend zwei Griffteile, die über ein Gelenk miteinander in Verbindung stehen. Eine Backe des Schneidteiles ist hohl ausgebildet, sodass sie die Versteifung aufnehmen kann. Die andere Backe weist eine Schneide auf, die im wesentlichen quer zur Längsachse der Zange steht. Die Länge der Backen sowie die Anordnung der Schneide in distaler Richtung werden vorzugsweise so gewählt, dass einerseits ein Schnitt im Zwischenwirbelbereich möglich ist, andererseits noch die notwendigen Abtrennkräfte sicher aufgebracht werden können. Dabei ist der elastische Körper so umschlossen, dass beim Abtrennen des nicht benötigten Spiralenendes keine fremden Gewebeteile mitgeschnitten werden. Natürlich können erfindungsgemäss auch Zangen Verwendung finden, deren Griffteile in einem Winkel zur Längsachse stehen.

In einer besonders bevorzugten Ausführung kann die Einbringvorrichtung auch ein Schneidinstrument beinhalten. Dies ist z.B. dadurch realisierbar, dass ein weiterer Stössel an seinem distalen Ende mit einer Schneidklinge versehen ist, die quer zur Längsachse der Vorrichtung verläuft. Die Schneidwirkung wird durch die distale Querschnittsverengung des Rohres, ähnlich wie beim Vorschub, erzeugt.

Das Implantat hat den Vorteil möglichst einfach im Aufbau zu sein und eine Stützwirkung gleichmässig über einen Grossteil seiner Fläche zu sichern. Das Implantat hat ferner den Vorteil nicht durch zusätzliche Mittel in Spiralform fixiert werden zu müssen, sondern kann mit vergleichsweise einfachen und sicher zu bedienenden Instrumenten eingebracht werden.

Im folgenden wird die Erfindung anhand eines besonders bevorzugten Ausführungsbeispieles im Zusammenhang mit den Zeichnungen näher erläutert.
- Fig. 1: zeigt schematisch und stark vergrössert den elastischen Körper in Form einer Spirale;
- Fig. 2: zeigt einen Querschnitt durch die Spirale nach Fig. 1;
- Fig. 3: zeigt in schematischer Darstellung eine Einführvorrichtung;
- Fig. 4: zeigt in schematischer Darstellung eine Abtrennvorrichtung und
- Fig. 5: zeigt schematisch eine Draufsicht auf die Abtrennvorrichtung gemäss Fig. 4.

Die Figuren handeln von einem Implantat, insbesondere Zwischenwirbelprothese, welches aus einem länglichen elastischen Körper besteht, der formelastisch ist und im kräftefreien Zustand die Form einer Spirale S annimmt. Die Spirale lässt sich durch Rückwärtsaufziehen in ein Einführinstrument einziehen, welches im Einführbereich nur unwesentlich grösser als der Querschnitt des länglichen elastischen Körpers ist, um durch eine kleine Oeffnung im anulus fibrosus den Innenraum einer Bandscheibe zu erreichen und die sich selbst aufwickelnde Spirale einzuschieben und abzutrennen, wenn der Innenraum gefüllt ist. Dies hat den Vorteil, dass unterschiedlich grosse Innenräume mit der gleichen Spirale gefüllt werden können.

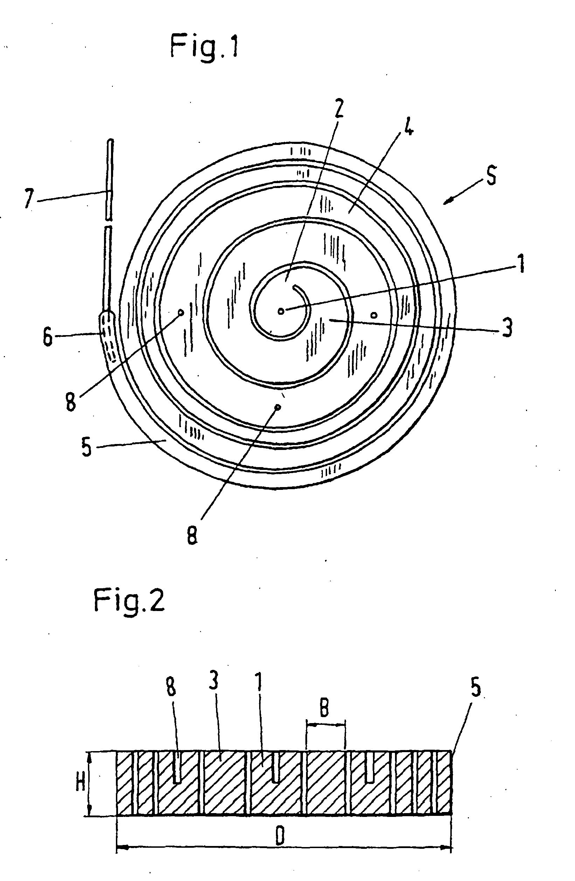

Fig. 1 zeigt das spiralförmige Implantat mit einem zentralen Mittelstück 1, das im wesentlichen die Form eines Zylinders aufweist. Daran schliesst seitlich ein dünnerer Bereich 2, der eine starke Abwinkelung und somit einen formschlüssigen Übergang zur daran anschliessenden Spiralwindung 3, im wesentlichen ohne Zwischenräume, ermöglicht.

Für den Fall, bei dem das Mittelstück entfällt empfiehlt es sich das innere Ende der Spiralwindung so auszubilden, dass bei Belastung keine Spannungspitzen auftreten können. Dies kann beispielsweise durch ein abgerundetes Ende erreicht werden.

Die an den Übergangsbereich anschliessenden Spiralwindungen sind formschlüssig und weisen eine konstante Breite im Bereich von 2 bis 5 mm auf, sodass ein Verkanten unter der zu erwartenden Belastung weitestgehend auszuschliessen ist. Das Implantat umfasst vorzugsweise 2-4 verkantungssichere Windungen auf. Daran schliesst ein Übergangsbereich 4 an, in dem die Breite des elastischen Körpers soweit reduziert wird, dass etwa im Bereich 5 von Fig. 1 ein leichtes Abtrennen möglich wird. Durch die Anzahl der Windungen wird die Spirale intra-operativ auf die gewünschte Grösse eingestellt. Die stützwirkung bezüglich Kippen ist in den äusseren schmalen Windungen als solche vermindert, jedoch in Verbindung mit den formstabileren inneren Windungen ausreichend gross.

Die Dicke des elastischen Körpers, der die Spirale bildet, kann aber auch stetig von innen her abnehmen, sodass kein besonders ausgebildeter Übergangsbereich notwendig ist. In Fig. 2 ist die Spirale im Querschnitt dargestellt. Sie hat einen Durchmesser im Bereich von 20 bis 30 mm, vorzugsweise im Bereich von 23 bis 25,5 mm. Die Höhe beträgt vorzugsweise 5-10 mm. Die Höhe kann auch in zentraler Richtung kontinuierlich zunehmen und im Zentrum maximal 10 mm annehmen. Die Höhe kann auch dem individuellen Verlauf der Wirbelflächen angepasst sein, was insbesondere bei Wirbelkörperdeformationen von Interesse sein kann.

An das freie Ende der Spirale 6 ist eine bandförmige Versteifung 7 fixiert, welche über das freie Ende hinausragt und als Verlängerung beim Hantieren der Spirale dienlich ist. Der Übergang soll möglichst glatt sein. Das Band soll in der Lage sein Schubkräfte zu übertragen und wird aus Biokompatibilitäts- und Sterilisationsgründen vorzugsweise aus Titan gefertigt. Seine Länge beträgt praktischer Weise etwa 20 cm und ermöglicht eine Führung des Abtrenninstrumentes bis zur Abtrennstelle an der Spirale.

Der elastische Körper besteht aus Kunststoff, etwa Polyurethan, wobei im kräftefreien Zustand die Spiralwindungen weitestgehend förmschlüssig zu liegen kommen. Die elastischen Eigenschaften des Materials müssen so sein, dass auch nach einem Ausrollen, wieder die Spiralform eingenommen wird. Die Deformationseigenschaften des Materials sollen denen der Bandscheibe weitestgehend ähnlich sein und im Bereich der Shore-Härte von 70A bis 90A, vorzugsweise um 80A, liegen.

Der elastische Körper kann zu seiner Herstellung aus dem Werkstoff ausgeschnitten werden, was insbesondere mit computergesteuerten Lasern möglich ist. Schneidvorgänge, die das Material thermisch nicht belasten sind bevorzugt, da so toxische Nebenprodukte vermieden werden und auch auf die elastischen Eigenschaften des Materials kein Einfluss genommen wird. Es muss nicht besonders erwähnt werden, dass das Material als solches sterilisierbar sein soll.

Um die Lage des Implantates intra- und post-operativ kontrollieren zu können, empfiehlt sich das Vorsehen von röntgen-positiven Markierungen in Form von Punkten 8 oder eines in den Abbildungen nicht dargestellten Fadens auf bzw. entlang und innerhalb des elastischen Körpers. Als Markermaterial kann Barium, Tantal oder Ähnliches Verwendung finden.

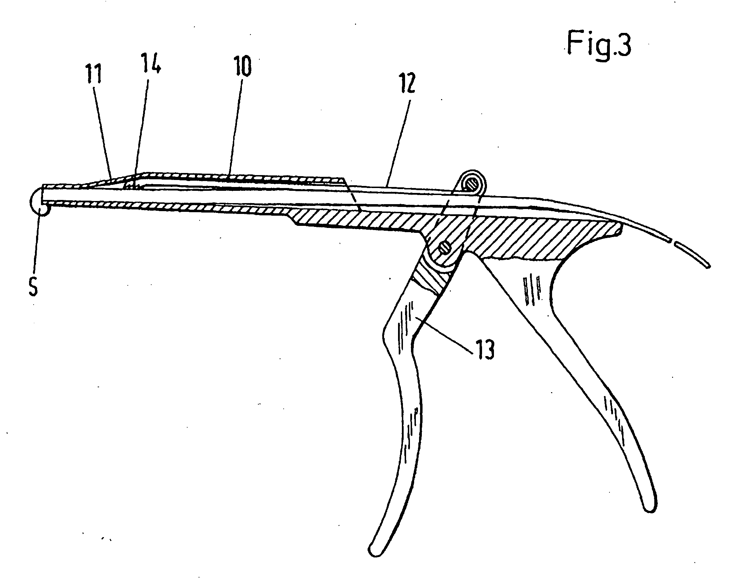

Die Vorrichtung zum Einbringen des elastischen Körpers ist in Fig. 3 näher dargestellt. Sie umfasst ein Vierkantrohr 10 zur Aufnahme der wenigstens soweit ausgerollten Spirale, dass ihr noch vorstehender Teil S problemlos durch die operativ geschaffene Öffnung in den Zwischenwirbelbereich eingebracht werden kann. Das Hohlrohr 10 weist vorzugsweise eine einseitige Querschnittsverengung 11 am distalen Ende auf, welche konisch oder schnabelartig ausgebildet ist. Das Rohr kann auch eine Krümmung aufweisen, die den Zugang zum Zwischenwirbelbereich von seitlich dorsal erleichtert und auf die Händigkeit des Benützers abgestellt ist. Die Krümmung kann aber auch nach caudal oder cranial gerichtet sein, je nach Anforderung.

Der Vorschub des Körpers kann auch von Hand erfolgen. Hierbei sind Griffmittel um das Rohr abzustützen hilfreich. Sie können koaxial mit dem Rohr in Form einer Verdickung ausgebildet sein oder radial zur Rohrachse verlaufen. Vorzugsweise sind jedoch Vorschubmittel in Form eines elastischen Stössels 12 vorgesehen, welche z.B. über einen Hebelmechanismus 13 betrieben werden können. Der Stössel weist an seinem distalen, in Wirkverbindung mit dem elastischen Körper oder mit der Versteifung stehenden Ende Mittel 14 auf, die einen Vorschub des Körpers erleichtern. Die Mittel können darin bestehen, die Oberfläche aufzurauhen, oder aber auch in zahnartigen Fortsätzen mit Vorzugsorientierung in distaler Richtung. Dadurch wird erreicht, dass die Fortsätze bei Vorschub des Stössels in den elastischen Körper oder in die Versteifung eingreifen und ihn/sie mit sich schieben, während sie bei Rückzug des Stössels über die Oberfläche nach hinten gleiten. Dabei ist ein kontrollierter Vorschub des elastischen Körpers möglich. Die einseitige Querschnittsverengung 11 des Vierkantrohres 10 bewirkt, dass der Stössel beim Vorschieben gleichzeitig nach unten gedrückt wird und so den Kraftschluss zwischen Stössel und Körper verstärkt. Dadurch dass der Stössel sehr weit vorne an der abgewickelten Spirale angreift, wird diese praktisch durch das Hohlrohr gezogen, sodass sie sich nicht unter ihrer Eigenspannung selbst blockiert, wie es bei Schub von hinten leicht der Fall sein könnte.

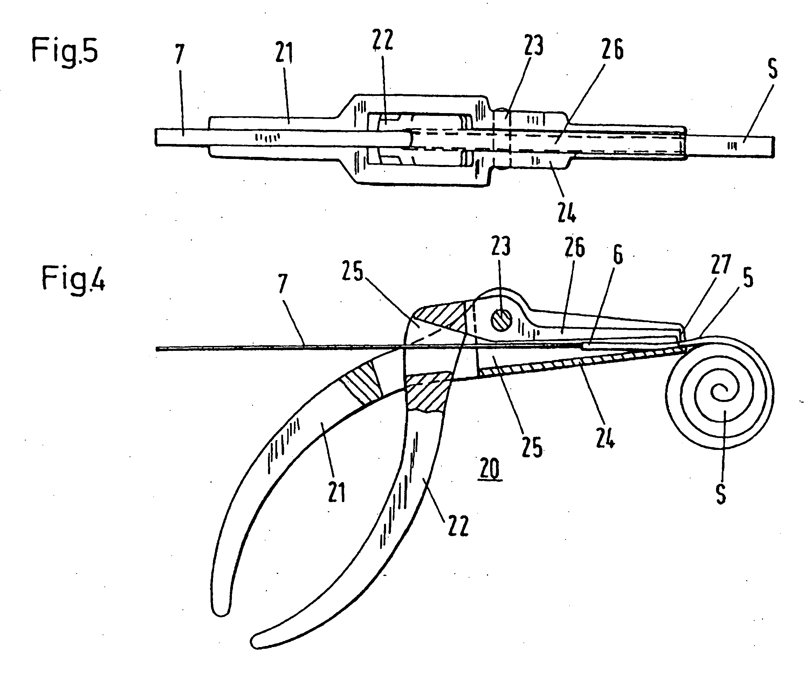

Fig. 4 und 5 zeigen eine Vorrichtung zum Abtrennen des überflüssigen Spiralenendes. Nachdem die Zwischenwirbelprothese implantiert worden ist, wird die Einführvorrichtung entlang der Versteifung 7, welche ausserhalb des Bandscheibenbereichs verbleibt, zurückgezogen und das Abtrenninstrument 20 aufgeschoben. Dieses Instrument weist vorzugsweise einen Wirkmechanismus ähnlich einer Zange auf, umfassend zwei Griffteile 21,22, die über eine Gelenk 23 miteinander in Verbindung stehen. Eine Backe 24 ist in Längsrichtung mit einer Ausnehmung 25 versehen, in die die Versteifung 7 und/oder der elastische Körper 5,6 aufgenommen werden kann. Die andere Backe 26 weist eine Schneide 27 auf, die im wesentlichen quer zur Ausnehmung 25 beweglich ist. Die Länge der Backen sowie die Anordnung der Schneide in distaler Richtung wird vorzugsweise so gewählt, dass einerseits ein Schnitt innerhalb des Zwischenwirbelbereiches möglich ist, andererseits noch die notwendigen Abtrennkräfte sicher aufgebracht werden können. Die Schneidklinge selbst kann als auswechselbare Klinge gestaltet sein. Natürlich können erfindungsgemäss auch Zangen Verwendung finden, deren Griffteile in einem Winkel zur Längsachse stehen.

Nachdem der elastische Körper auf die gewünschte Länge gekürzt worden ist, wird die Abtrennvorrichtung zusammen mit der Versteifung 7 zurückgezogen. Das Implantat nimmt aufgrund seiner formstabilen Eigenschaften die Spiralform an. Das Implantat behält auch unter Belastung seine Spiralform bei, ohne dass eine besondere Fixierung des freien Endes notwendig wäre.

Das zuvor beschriebene Verfahren zum Einbringen eines spiralförmigen Implantates in einen Hohlraum, kommt vorteilhafter Weise auch zu Lehr-, Übungs-, und Demonstrationszwecken zur Anwendung.

## Patentansprüche

1. Zwischenwirbelimplantat, insbesondere die Form einer Zylinderscheibe annehmend, bestehend aus einem länglichen, einteiligen und elastischen Körper aus Kunststoff mit einer Shore-Härte im Bereich von 70A bis 90A, beispielsweise Polyurethan, mit Deformationseigenschaften, die denen einer Bandscheibe weitgehend ähnlich sind, wobei der Körper formelastisch ist und im kräftefreien Zustand die Form einer Spirale (S) annimmt, wobei die Breite (B) des Körpers im Inneren der Spirale größer ist als bussen, wobei die Breite stetig von innen her abnimmt oder die Breite im Inneren der Spirale an das sich ein die Breite reduzierender Übergangsbereich zu äußeren schmalen Spiralwindungen anschließt, konstant ist, und wobei das Implantat dadurch erhalten wird, dass die Spirale im Spritzgussverfahren hergestellt wird, wobei eine schneckenförmige Gussform im Spiralinneren angegossen wird, damit nach einem Ausrollen der Spirale im kräftefreien Zustand die Spiralwindungen weitestgehend formschlüssig um ein zentrales Mittelstück zu liegen kommen.

2. Implantat nach Anspruch 1, **dadurch gekennzeichnet, dass** der Körper im wesentlichen einen rechteckigen Querschnitt (H,B) aufweist, dessen Höhe (H) dem zu ersetzenden Zwischenwirbelbereich entspricht.

3. Implantat nach Anspruch 1, **dadurch gekennzeichnet, dass** die Spirale eine ebene Grundfläche und/oder eine ebene Deckfläche aufweist.

4. Implantat nach Anspruch 1, **dadurch gekennzeichnet, dass** die Spirale wenigstens eine nach aussen gewölbte Grund- und/oder Deckfläche aufweist.

5. Implantat nach Anspruch 2, **dadurch gekennzeichnet, dass** das Höhen zu Breitenverhältnis des Querschnitts des formelastischen Körpers wenigstens im Zentrum der Spirale so ausgebildet ist, dass der Körper unter Belastung nicht verkantet oder kippt.

6. Implantat nach Anspruch 1, **dadurch gekennzeichnet, dass** die Spirale in ihrem Zentrum ein annähernd zylindrisches Mittelstück (1) aufweist, an dem seitlich der formelastische Körper, gegebenenfalls über ein Übergangsglied (2) geringerer Breite, ansetzt, um im kräftefreien Zustand eine formschlüssige Spirale im wesentlichen ohne Zwischenräume zu erzeugen.

7. Implantat nach Anspruch 1, **dadurch gekennzeichnet, dass** der Körper aus sterilisierbarem Material besteht, wobei das Material vorzugsweise Polyurethan, oder ein Polyurethan-Silicongemisch ist und eine Shore-Härte im Bereich von etwa 80A aufweist.

8. Implantat nach Anspruch 1, **dadurch gekennzeichnet, dass** der Körper in Spiralform einen Aussendurchmesser (D) annimmt, der den Raum innerhalb des annulus fibrosus im wesentlichen vollständig ausfüllt, wobei der Aussendurchmesser im Bereich zwischen 20 und 30 mm, vorzugsweise im Bereich zwischen 23 und 25,5 mm liegt, sowie vorzugsweise eine Höhe von 5 bis 10 mm aufweist und die Spirale mindestens 3, vorzugsweise 5 Windungen aufweist.

9. Implantat nach Anspruch 1, **dadurch gekennzeichnet, dass** am freien Ende (6) der Spirale zusätzlich Versteifungsmittel (7), vorzugsweise in Form eines Bandes, vorgesehen sind, über welches Schubkräfte übertragen werden können, wobei das Band aus Kunststoff oder Metall, vorzugsweise aus Titan, gefertigt ist.

10. Impantat nach Anspruch 1, **dadurch gekennzeichnet, dass** Röntgenmarker (8), vorzugsweise in Form von Kugeln oder Fäden vorgesehen sind.

## Claims

1. Intervertebral implant, in particular adopting the shape of a cylindrical disk, consisting of an elongated, one-part and elastic body made of plastic having a Shore hardness in the range from 70A up to 90A, for example of polyurethane, having deformation properties which are largely similar to those of an intervertebral disc, with the body being elastic in shape and adopting the shape of a spiral (S) in the force-free state, wherein the width (B) of the body is greater at the inside of the spiral than at the outside, wherein the width reduces constantly from the inside or the width is constant in the interior of the spiral which is adjoined by a transition region to outer narrow spiral windings reducing the width, and wherein the implant is obtained in that the spiral is manufactured in the injection moulding process, with injection moulding being effected at the inside of the spiral in a spiral mould so that, after a rolling out of the spiral, the spiral windings come to rest in a very largely shape matched manner around a central middle piece in the force-free state.

2. Implant in accordance with claim 1, **characterised in that** the body has a substantially rectangular cross-section (H, B) whose height (H) corresponds to the intervertebral region to be replaced.

3. Implant in accordance with claim 1, **characterised in that** the spiral has a planar base surface and/or a planar top surface.

4. Implant in accordance with claim 1, **characterised in that** the spiral has at least one outwardly arched base surface and/or top surface.

5. Implant in accordance with claim 2, **characterised in that** the height to width ratio of the cross-section of the body with an elastic shape is formed at least at the centre of the spiral such that the body does not cant or tilt under load.

6. Implant in accordance with claim 1, **characterised in that** the spiral has an approximately cylindrical middle piece (1) at its centre to which the body with an elastic shape is joined at the side, optionally via a transition member (2) of smaller width, in order to produce a shape-matched spiral substantially without intervening spaces in the force-free state.

7. Implant in accordance with claim 1, **characterised in that** the body consists of sterilisable material, with the material preferably being polyurethane or a polyurethane/silicone mixture and having a Shore hardness in the range of approximately 80A.

8. Implant in accordance with claim 1, **characterised in that**, in spiral form, the body adopts an external diameter (D) which substantially completely fills up the space within the annulus fibrosus, with the external diameter lying in the range between 20 and 30 mm, preferably in the range between 23 and 25.5 mm, and preferably having a height from 5 to 10 mm and with the spiral having at least 3 windings, preferably 5 windings.

9. Implant in accordance with claim 1, **characterised in that** stiffening means (7), preferably in the form of a band, are additionally provided at the free end (6) of the spiral via which shear forces can be transmitted, with the band being made of plastic or of metal, pref erably of titanium.

10. Implant in accordance with claim 1, **characterised in that** X-ray markers (8) are provided, preferably in the form of spheres or threads.

## Revendications

1. Implant intervertébral, prenant en particulier la forme d'un disque cylindrique, composé d'un corps allongé, d'une seule pièce et élastique à base de matière plastique avec une dureté Shore dans la plage de 70A à 90A, par exemple du polyuréthane, avec des propriétés de déformation qui sont largement similaires à celles d'un disque intervertébral, le corps étant élastique au niveau de la forme et prenant, en l'absence de forces, la forme d'une spirale (S), la largeur (B) du corps à l'intérieur de la spirale étant supérieure à celle de l'extérieur, la largeur diminuant constamment à partir de l'intérieur ou bien la largeur étant constante à l'intérieur de la spirale, auquel se raccorde une zone de transition réduisant la largeur avec des spires de spirale étroites extérieures, et l'implant étant obtenu par le fait que la spirale est fabriquée selon le procédé de moulage par injection sous pression, un moule en forme d'escargot étant coulé à l'intérieur de la spirale, afin que, après un déroulement de la spirale en l'absence de forces, les spires de spirale viennent se positionner le plus largement possible par complémentarité de formes autour d'une partie centrale.

2. Implant selon la revendication 1, **caractérisé en ce que** le corps comprend essentiellement une section (H, B) rectangulaire, dont la hauteur (H) correspond au secteur intervertébral à remplacer.

3. Implant selon la revendication 1, **caractérisé en ce que** la spirale présente une surface de base plane et/ou une surface de recouvrement plane.

4. Implant selon la revendication 1, **caractérisé en ce que** la spirale comprend au moins une surface de base et/ou de recouvrement bombée vers l'extérieur.

5. Implant selon la revendication 2, **caractérisé en ce que** le rapport hauteur/largeur de la section du corps élastique au niveau de la forme est réalisé au moins au centre de la spirale, de sorte que le corps sous charge ne penche ni ne bascule.

6. Implant selon la revendication 1, **caractérisé en ce que** la spirale comprend en son centre une pièce centrale (1) approximativement cylindrique, sur lequel le corps élastique au niveau de la forme s'applique latéralement, le cas échéant via un élément de transition (2) de largeur inférieure, afin de créer en l'absence de forces une spirale complémentaire au niveau de la forme essentiellement sans espaces intermédiaires.

7. Implant selon la revendication 1, **caractérisé en ce que** le corps est à base de matériau stérilisable, le matériau étant de préférence du polyuréthane ou un mélange de polyuréthane et silicone et présentant une dureté Shore dans la plage proche de 80A.

8. Implant selon la revendication 1, **caractérisé en ce que** le corps en forme de spirale prend un diamètre extérieur (D), qui remplit pratiquement complètement l'espace à l'intérieur de l'anneau fibreux, le diamètre extérieur se situant dans la plage entre 20 et 30 mm, de préférence entre 23 et 25,5 mm, et présente de préférence une hauteur de 5 à 10 mm et la spirale présente au moins 3_{;} de préférence 5 spires.

9. Implant selon la revendication 1, **caractérisé en ce qu'**à l'extrémité (6) libre de la spirale sont prévus des moyens de renforcement (7) supplémentaires, de préférence sous la forme d'une bande, par laquelle des forces de poussée peuvent être transmises, la bande étant fabriquée en matière plastique ou en métal, de préférence en titane.

10. Implant selon la revendication 1, **caractérisé en ce que** des marqueurs radiographiques (8) sont prévus de préférence sous forme de sphères ou de fils.
